# EUROPEAN PATENT SPECIFICATION

(11) **EP 1 251 883 B1**
(45) Date of publication and mention of the grant of the patent: **27.10.2004**
(21) Application number: 00914797.6
(22) Date of filing: 02.03.2000
(51) Int. Cl.: A61L 9/014, A61L 9/12

(54) **APPARATUS AND METHOD FOR DEODORIZING AIR**
VORRICHTUNG UND VERFAHREN ZUM DESODORIEREN VON LUFT
APPAREIL ET PROCEDE DE DESODORISATION DE L'AIR

(30) Priority: 04.02.2000 US 3011
(43) Date of publication of application: 30.10.2002
(73) Proprietor: THE PROCTER & GAMBLE COMPANY, Cincinnati, Ohio 45202 (US)
(72) Inventor: PAINTER, Jeffrey, Donald, Loveland, OH 45140 (US); KVIETOK, Frank, Andrej, Cincinnati, OH 45208 (US); TRINH, Toan, Maineville, OH 45039 (US)
(74) Representative: Brooks, Maxim Courtney
(86) International application number: PCT/US2000/005403
(87) International publication number: WO 2001/056622

(56) References cited:
- EP-A- 0 311 454
- DE-A- 3 002 409
- DE-A- 3 640 953
- US-A- 3 972 678
- US-A- 4 235 750
- US-A- 5 772 738
- DATABASE WPI Section Ch, Week 199151 Derwent Publications Ltd., London, GB; Class A88, AN 1991-373442 XP002148972 & JP 03 251253 A (DAISO CO LTD), 8 November 1991 (1991-11-08)
- DATABASE WPI Section Ch, Week 199918 Derwent Publications Ltd., London, GB; Class D22, AN 1999-208546 XP002148971 & JP 11 047256 A (KAWAI MUSICAL INSTR MFG CO), 23 February 1999 (1999-02-23)
- DATABASE WPI Section Ch, Week 199524 Derwent Publications Ltd., London, GB; Class D14, AN 1995-181605 XP002148973 & JP 06 288672 A (GOLDSTAR CO LTD), 18 October 1994 (1994-10-18)

## Description

### Field of the invention

The present invention relates a method for removing malodor from the air. Such method is useful for example for storing and preserving food in closed compartments such as refrigerators.

### Background

Nowadays, refrigerators have become a common appliance in virtually every household and typically are used for storage and preservation of food, in particular of fresh food such as fruits, vegetables, dairy products, and the like. It is desirable to keep the food items fresh and free of contaminated odor as long as possible in the refrigerator.

It is a well known problem that many food items tend to release malodors into the air which are then captured in the limited air space in a refrigerator. Not only are these malodors unpleasant and offensive to the user of the refrigerator, they can also have a negative impact on the quality of other foods in the refrigerator. For example, it is known that some foods emit strong odors (e.g. fish, boiled eggs, onions, etc.) and that these odors can transfer to other nearby foods and hurt the taste and freshness of those foods. A common example is the transfer of odors into an open container of orange juice or of milk resulting in a noticeable degradation in their taste. It is also well known that malodors from some vegetables (onions, garlic) can transfer to other foods stored within a vegetable drawer. This problem is aggravated when the vegetable drawer is sealed such that there is very little air exchange with the larger compartment of the refrigerator (herein referred to as the "fresh food compartment") and when vegetables have been cut or are stored without any outer wrapping. This problem of odor transfer is particularly acute in the case of ice cubes where odors from the fresh food compartment of the refrigerator can be transferred to the ice in the freezer compartment of the refrigerator. This is especially true in the case of refrigerators in which there is air exchange between the fresh food and freezer compartments, and especially in the case of refrigerators with built-in ice-makers.

A common attempt to solve this odor contamination problem is the use of baking soda, especially in the refrigerators. However, this static method is not very effective, because the bulk of the baking soda is not exposed to the contaminated air, and the air movement is minimal. There exist in the art devices for deodorizing confined spaces such as disclosed for example in U.S. patent No. 5, 403,548 and in U.S. patent No 5,772,959. However, these devices as such fail to be capable of deodorizing those complexly structured confined spaces which comprise portions which are not reached by normal air convection. In these portions which include for example drawers for fresh fruits and vegetables in a refrigerator, malodors can be trapped and hence cannot be reached by deodorizing devices placed in the main compartment.

It is therefore an object of the present invention to provide a method for deodorizing confined spaces which overcomes the disadvantages of the prior art.

It is a further object of the present invention to provide a method for deodorizing confined spaces which is capable of deodorizing confined spaces comprising portions which are not accessible to normal air convection to an extent allowing for deodorization of those portions.

### Summary of the invention

It is one aspect of the present invention to provide a method for deodorizing air in a confined space said confined space comprising a compartment separate from the remainder of said confined space comprising the steps of
- providing a first member comprising a first filter member wherein said first member is a forced air filter member further comprising an air moving member
- providing a second member comprising a second filter member wherein said second member is a passive member
- positioning the first member inside the remainder of the confined space
- positioning the second member inside the compartment independent from the position of the first member
- removing malodor molecules from the air of the confined space by adsorbing the molecules onto the surfaces of the first filter member and onto the surfaces of the second filter member.

### Detailed description of the invention

The method for deodorizing air in confined spaces of the present invention is suitable for refrigerators, closets, and the like.

Confined spaces often have complex structures so that normal air convection does not reach every comer of the confined space. Such complex structures for example include separate compartments such as drawers or hollow elements inside the confined space. Therefore, it is insufficient to deodorize such confined spaces with only a single device, even a forced air device having a fan.

With the method of the present invention it is possible to deodorize those portions of the confined space to which are not sufficiently accessible to normal air convection. This is achieved by utilizing more than one filter member whereby each of those filter members can be positioned independent of each other whereby one of the filter members optionally can be used while connected to an air moving member.

Suitable filter members for the method of the present invention include passive filter members and forced air filter members. The term "passive filter member" as used herein refers to those filter members which only rely on air convection and on diffusion to bring malodors within reach of the filter media in the member. The term "forced air filter member" as used herein refers to those filter members which attached to a forced air moving member which draws air into the device through a filter member containing a filter media and increases air flow through the filter media above that achieved through normal air convection in the confined space. As used within this disclosure, a forced air filter member consists of a filter member and an air moving member. A filter member suitable for the forced air filter members can be a passive filter member according to the above definition.

A confined space for which the method of the present invention is particularly suitable comprises a compartment which is within the confined space but which is separated from the remainder of the confined space. In this case, one of the filter members can be placed in the separate compartment and another filter element can be placed in the remainder of the confined space. With the method of the present invention it is therefore possible to deodorize all compartments being comprised in a confined space such as a refrigerator (which has enclosed compartments for vegetables, meats, etc.), a closet (which has shoe storage closets, clothes storage containers, etc.), or the like. In particular when the separate compartments differ in size, it can be beneficial to place a forced air filter member in the larger compartment and a passive filter member in the smaller compartment.

Deodorization of the air to remove malodors can be achieved by adsorbing the molecules constituting a malodor onto a surface of a filter member. The term "adsorption" is well defined in the art and refers to the adherence of molecules to surfaces which effectively reduces the mobility of these molecules to the two dimensions of the surface. Those molecules remaining in the air will then diffuse so that further molecules come into contact with the surface and subsequently will be adsorbed. Consequently, most of the malodor molecules will travel into the proximity of one of the surfaces at some point in time so that finally most of the malodor will be removed from the air.

A filter member suitable to be used in the first member and/or in the second member of the present invention can comprise activated carbon for the adsorption. Activated carbon is known to be a very effective filter medium due to its high specific surface area. Whilst activated carbon is very effective as such, the filter member of the present invention can further comprise agents supported on the activated carbon to specifically remove certain malodor molecules such as those comprising S atoms or N atoms. A wide variety of activated carbon based filter media is known in the art. Preferably, the filter members of the present invention comprise at least about 2 grams, more preferably at least about 5 grams, and most preferably at least about 10 grams of activated carbon. Preferably, the filter members of the present invention comprise less than about 100 grams, more preferably less than about 50 grams, yet more preferably less than about 40 grams, and even more preferably less than about 30 grams of activated carbon. Alternatively, the filter member of the present invention can comprise a filter medium capable of removing ethylene from the air, such as a filter medium comprising potassium permanganate.

The filter members of the present invention comprise an air inlet, an air outlet, and an air flow path through the filter member from the air inlet to the air outlet. The filter medium is disposed in the filter member of the present invention such that it comes into contact with the air flowing along the air flow path. The filter medium can be arranged as a flow by filter or as a flow through filter. The filter member of the present invention can comprise a support for the filter medium for example in the form of a foam, a nonwoven material or a woven material.

The deodorization of the air in the forced air device of the present invention is enhanced by increasing the air flow through the filter member by means of an air moving member. The air moving means typically moves at least about 10 ml of air per second through the air inlet into the device, preferably at least about 50 ml/s, more preferably at least about 100 ml/s, even more preferably at least about 200 ml/s, and still more preferably at least about 300ml/s. Even higher air flow rates are preferred for achieving better results, and can be achieved through using higher capacity power sources, through rechargeable batteries which are removed and recharged at more frequent intervals, or through use of AC electrical power available in a refrigerator or through common household power outlets. There are known in the art a wide variety of suitable air moving members such as for example fans and blowers. A particularly suitable fan is a centrifugal fan. A suitable member for driving the fan is a small motor, for example a DC motor available from MABUCHI MOTOR CO.,LTD., Japan, under the designation of RF-330TK. The air moving members of the present invention are powered electrically. Many electrical power sources could be imagined including domestic AC electrical power or power from a static power supply. Alternatively and preferably electrical power can be supplied by means of a battery, preferably a dry alkaline cell battery, or a rechargeable battery. Any replaceable power supply is designed to last at least about one week, preferably at least about one month, more preferably at least about two months, yet more preferably at least about three months, even more preferably at least about four months.

A forced air device will enable adsorption of more malodors to happen more quickly than a passive device by increasing the rate of air contact with the adsorbing media. As such, a forced air device as described above is suitable for use in the large area of a confined space (e.g. the fresh food compartment of a refrigerator, a closet, etc.) as it has the ability to move the relatively large volume of air in these spaces, typically 50 to 800 liters, through the filter media. A passive device is suitable for use in the smaller compartments within a confined space (vegetable drawers, shoe storage containers, etc.) where diffusion alone is effective at moving air to the filter media, and where the air is not accessible to the forced air device.

Preferably, filter elements and air moving member are designed such that any air that is drawn into the air moving member is forced to flow through the attached filter member before penetrating into the air moving member. By this construction, the mechanical complexity of the apparatus of the present invention is reduced since only one connection between filter member and air moving member is needed.

## Claims

1. A method for deodorizing air in a confined space said confined space comprising a compartment separate from the remainder of said confined space comprising the steps of
-- providing a first member comprising a first filter member wherein said first member is a forced air filter member further comprising an air moving member
-- providing a second member comprising a second filter member wherein said second member is a passive member
-- positioning said first member inside the remainder of said confined space
-- positioning said second member inside said compartment independent from the position of said first member
-- removing malodor molecules from the air of said confined space by adsorbing said molecules onto the surfaces of said first filter member and onto the surfaces of said second filter member.

2. A method for deodorizing air in a confined space according to claim 1 wherein
said first filter member and/or second filter member comprises activated carbon.

3. A method for deodorizing air in confined spaces according to claim 1 wherein
said air moving member comprises a fan and an electrical drive member driving the fan.

4. A method for deodorizing air in a confined space according to claim 1 wherein
said confined space is the confined space of a refrigerator.

## Patentansprüche

1. Verfahren zum Desodorisieren von Luft in einem begrenzten Raum, wobei der begrenzte Raum eine Kammer umfasst, welche von dem Rest des begrenzten Raums getrennt ist, umfassend die Schritte
- Vorsehen eines ersten Teils, umfassend ein erstes Filterelement, wobei das erste Teil ein Gebläseluftfilterelement ist, das weiterhin ein Luftbewegungselement umfasst,
- Vorsehen eines zweiten Teils, umfassend ein zweites Filterelement, wobei das zweite Teil ein passives Element ist,
- Positionieren des ersten Teils innerhalb des Restes des begrenzten Raums,
- Positionieren des zweiten Teils innerhalb der Kammer unabhängig von der Position des ersten Teils,
- Entfernen von Übelgeruchsmolekülen aus der Luft des begrenzten Raums durch Adsorbieren der Moleküle auf den Oberflächen des ersten Filterelements und auf den Oberflächen des zweiten Filterelements.

2. Verfahren zum Desodorisieren von Luft in einem begrenzten Raum nach Anspruch 1, wobei das erste Filterelement und/oder das zweite Filterelement Aktivkohle umfasst.

3. Verfahren zum Desodorisieren von Luft in einem begrenzten Raum nach Anspruch 1, wobei das Luftbewegungselement einen Ventilator und ein elektrisches Antriebselement, das den Ventilator antreibt, umfasst.

4. Verfahren zum Desodorisieren von Luft in einem begrenzten Raum nach Anspruch 1, wobei der begrenzte Raum der begrenzte Raum eines Kühlschranks ist.

## Revendications

1. Procédé pour désodoriser l'air d'un espace confiné, ledit espace confiné comprenant un compartiment séparé du reste dudit espace confiné, comprenant les étapes consistant à :
- prévoir un premier élément comprenant un premier élément formant filtre, ledit premier élément étant un élément formant filtre à ventilation forcée comprenant, en outre, un élément de déplacement d'air,
- prévoir un deuxième élément comprenant un deuxième élément formant filtre, ledit deuxième élément étant un élément passif,
- placer ledit premier élément à l'intérieur du reste dudit espace confiné,
- placer ledit deuxième élément à l'intérieur dudit compartiment indépendant de la position dudit premier élément,
- enlever les molécules malodorantes de l'air dudit. espace confiné en adsorbant lesdites molécules sur les surfaces dudit premier élément formant filtre et sur les surfaces dudit deuxième élément formant filtre.

2. Procédé pour désodoriser l'air d'un espace confiné selon la revendication 1, dans lequel :
le(s)dit(s) premier élément formant filtre et/ou deuxième élément formant filtre comprennent(comprend) du charbon activé.

3. Procédé pour désodoriser l'air d'un espace confiné selon la revendication 1, dans lequel :
ledit élément de déplacement d'air comprend un ventilateur et un organe d'entraînement électrique qui entraîne le ventilateur.

4. Procédé pour désodoriser l'air d'un espace confiné selon la revendication 1, dans lequel
ledit espace confiné est l'espace confiné d'un réfrigérateur.
